# EUROPEAN PATENT APPLICATION

(11) **EP 3 158 933 A1**
(43) Date of publication of application: **26.04.2017**
(21) Application number: 16195331.0
(22) Date of filing: 24.10.2016
(51) Int. Cl.: A61B 5/11, A61B 5/16, A61B 5/00, G06F 19/00

(54) **FUNCTIONAL LEARNING DEVICE, SYSTEM, AND METHOD**

(30) Priority: 22.10.2015 US 201514920840
(71) Applicant: Activarium, LLC, Excelsior, MN 55331 (US)
(72) Inventor: LOWERY, Elizabeth, Excelsior, MN 55331 (US); MANE, Nelson, Odessa, FL 33556 (US)
(74) Representative: Boden, Keith McMurray

(57) **Abstract**

A device, system, and method that supplement learning and cognitive skills in individuals, such as children or adults, with neurodevelopmental learning disorders or age-related cognitive decline. More specifically, the disclosed invention relates to a whole body fitness program to integrate motion, motor-sensory learning, and vision to change the brain's neuronal pathways and make learning and cognitive skills easier.

## Description

### FIELD OF THE DISCLOSURE

The disclosed invention relates to a device, system, and method that supplement learning and cognitive skills in individuals, such as children or adults, with neurodevelopmental learning disorders or age-related cognitive decline. More specifically, the disclosed invention relates to a whole body fitness program and Bluetooth-enabled glasses to integrate motion, motor-sensory learning, and vision to change the brain's neuronal pathways and make learning and cognitive skills easier.

### BACKGROUND OF THE INVENTION

Current research has provided strong evidence that the brain has an inherent neuroplasticity that allows for re-wiring of neural pathways during the lifetime of an individual. For this reason, neurological disorders such as ADD, ADHD, autism, Asperger's, dyslexia, Alzheimer's, Dementia, Parkinson's and other sensory and processing disorders are no longer considered incurable, lifelong challenges. Similarly, brain tumors and concussions are also manageable since the brain can be re-wired so that an alternative pathway is created when the original pathway has either been removed or compromised. The above-described kinds of neurodevelopmental disorders are believed to be variations of one neurological condition, commonly referred to as hemispheric imbalance, functional disconnection, or under-connectivity. Although the disorders commonly show a combination of behavioral, social, academic, physical, and sensory symptoms, the disorders stem from a specified functional weakness in either the bottom to the top or the left or right side hemispheres of the brain. When a child has a learning disability, the disability is often triggered by a lower level functioning system that is miswired or not connected at all. A child with issues in reading, spelling, math, attention, focus, or behavior most likely is affected by a functional weakness in visual/motor skills, visual/auditory memory, left to right visual tracking, or any other number of lower-level brain function combinations. Some commonly known left hemisphere disorders include dyslexia, dysgraphia, and language processing disorders. Some commonly known right hemisphere disorders include ADD/ADHD, autism, Asperger's, non-verbal learning disorders, Tourette's, and OCD.

In the past, parents have been given long, arduous plans for incorporating years of time-consuming treatment into their children's lives. Further, little gains resulted after several years of therapy, tutors, and specialists. Therefore, new technology is needed that is more time-efficient and more beneficial to individuals with neurodevelopmental disorders and age-related cognitive decline.

### SUMMARY OF THE INVENTION

The disclosed invention includes systems, methods and devices that supplement learning in individuals, such as children and adults, with neurodevelopmental learning disorders or age-related cognitive decline. More specifically, the system includes brain function assessment, brain fitness training activities, motion-sensing technology, data collection methods, data analysis methods, communication capabilities, Internet capabilities, and an online community platform that can engage and offer support to users. In one embodiment, the system includes Bluetooth-enabled glasses that stimulate specific hemispheres of the brain and coordinate music play when a user is completing an activity.

### BRIEF DESCRIPTION OF THE DRAWINGS

FIG. 1 illustrates cognitive brain functions from most basic to most complex, wherein some brain functions are considered upper-level brain functions and others are considered lower-level brain functions.
FIG. 2 illustrates the core components of the disclosed system and method.
FIG. 3 illustrates the first of two parts of the assessment and corresponding activities flowchart.
FIG. 4 illustrates the second of two parts of the assessment and corresponding activities flowchart.
FIG. 5 illustrates how a user with a left hemisphere deficit can interact with the disclosed system and method.
FIG. 6 illustrates how a user with a right hemisphere deficit can interact with the disclosed system and method.
FIG. 7 illustrates how the brain processes visual stimuli.
FIG. 8 illustrates how colors can be used to stimulate different brain hemispheres.
FIG. 9 illustrates how blocking half of each side of the eye and using a specific color can stimulate a specific hemisphere of the brain.
FIG. 10 illustrates Bluetooth-enabled glasses with red/blue lenses according to one embodiment of the disclosed system.
FIG. 11 is a schematic block diagram depicting an example computing system used in accordance with one embodiment of the present invention.

### DETAILED DESCRIPTION

Various embodiments will be described in detail with reference to the drawings, wherein like reference numerals represent like parts and assemblies throughout the several views. Reference to various embodiments does not limit the scope of the claims attached hereto. Additionally, any examples set forth in this specification are not intended to be limiting and merely set forth some of the many possible embodiments for the appended claims. It is understood that various omissions and substitutions of equivalents are contemplated as circumstances may suggest or render expedient, but these are intended to cover applications or embodiments without departing from the spirit or scope of the claims attached hereto. Also, it is to be understood that the phraseology and terminology used herein are for the purpose of description and should not be regarded as limiting.

The disclosed invention, in one embodiment, is designed to find the functional imbalance, or weak area or areas, in the brain through assessment of the different levels of brain function using a compared weighting system that compares left hemisphere to right hemisphere and also compares the top hemisphere to the bottom. The system can then produce a solution, such as one or more series of interactive activities, that is specific to the user and that targets the functional imbalance or weaknesses. Under the disclosed system, training activities modify as the user continues to complete them, ensuring continuous brain activation. As the user adapts to the activity, the system modifies the activity by increasing the difficulty and, therefore, brain activation.

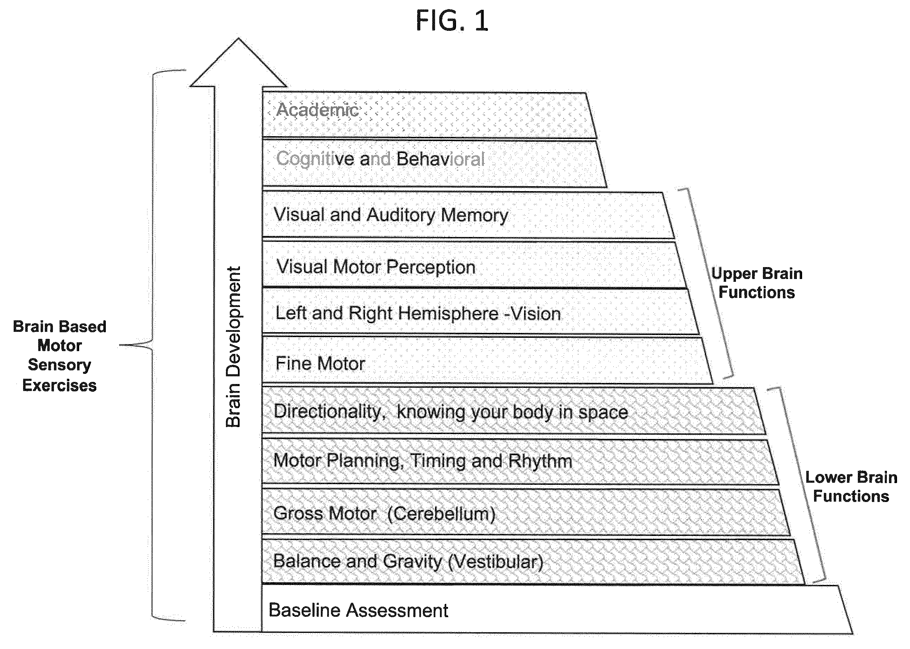

The levels, as illustrated in FIG. 1, from lowest to highest that are included in assessment and training can include, but are not limited to, vestibular, cerebellum, motor planning and timing, directionality, fine motor, visual motor perception, and visual and auditory memory. After determining any imbalances or weaknesses, the disclosed invention can then correct those imbalances or weaknesses through training exercises and integrate those corrected imbalances or weaknesses into later exercises or daily routines. The system is capable of taking an assessment or evaluation at a first point in time and then measuring gaps and improvements between that assessment and a user's exercise to determine if the user is, in fact, improving and, if so, it can determine future exercises for the user.

Several training activities can be used to improve any imbalance of the brain of an individual. The training exercises can target and strengthen the under-functioning hemisphere of the brain through incorporation of activities focused on balance, motor, timing and rhythm, directionality, fine motor skills, vision, auditory, and memory.

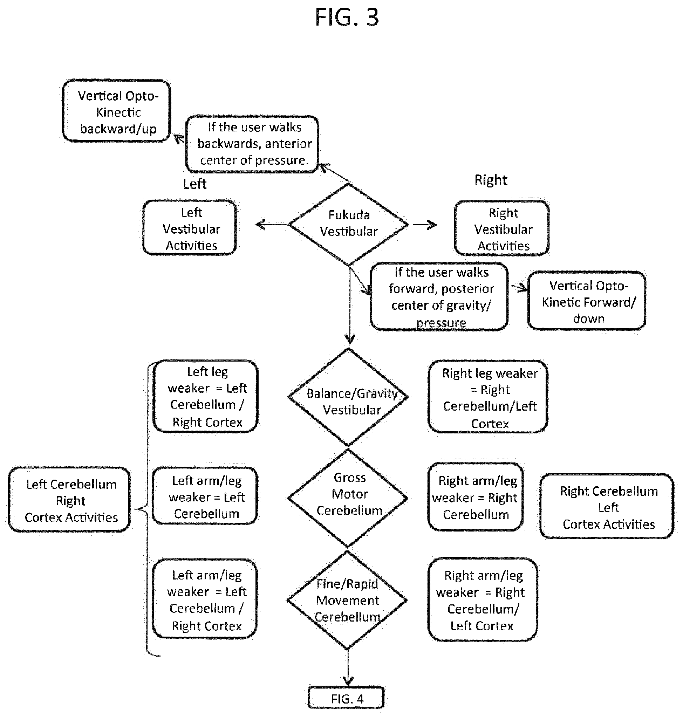

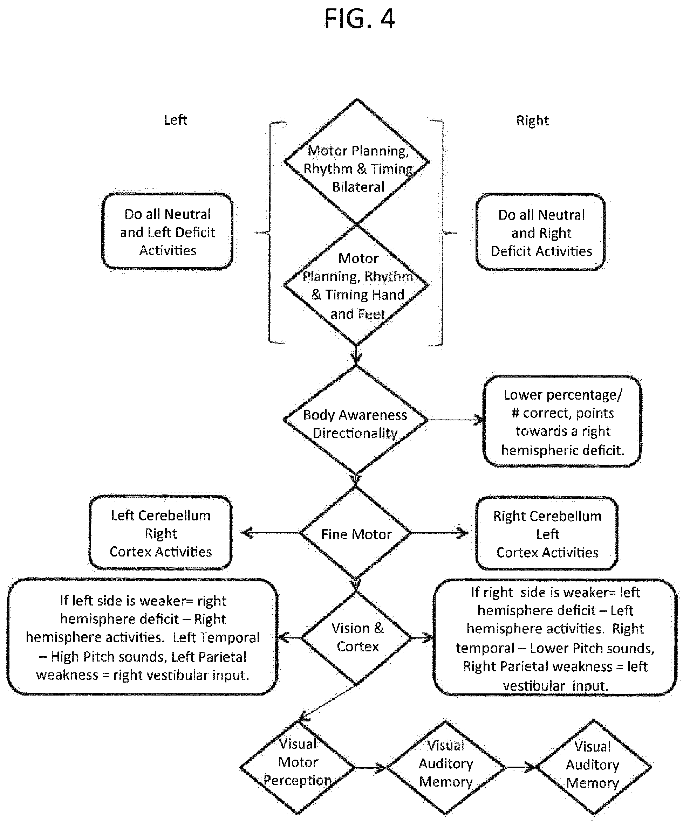

In one embodiment, as illustrated in FIGS. 3 and 4, the disclosed system tests the user's lower-level and higher-level brain functionality and determines if and where a user needs improvement. More specifically, the disclosed system can first assess a baseline, next assess various lower brain functions, and then assess upper brain functions. Lower brain functions include balance and gravity (vestibular), gross motor (cerebellum), motor planning, timing and rhythm, and directionality. Upper brain functions include fine motor, left and right hemisphere vision, visual motor perception, and visual and auditory memory.

Therefore, the user may initially have a baseline assessment completed that determines the user's heart rate and completes a face scan. Next, the user's vestibular brain functions can be assessed. This can be completed, for example, by implementing the Fukuda Stepping Test for Vestibular Function and/or a balance test. The user gross motor and cerebellum assessment can use a finger to nose test, a figure eight's test, and/or a pancake hands test. The motor planning, timing, and rhythm assessment can use, for example, a cross crawl test and/or a dance sequence test. The remaining assessments all have additional tests that can be completed for a proper assessment of a user's brain functionality.

The system then delivers a series of activities across all the functional areas targeted for improvement. For example, if the system determines that a user has a balance and gravity (vestibular) weakness, the system can deliver a series of training exercises or a series of activities such as hopscotch and/or balance/yoga.

In an alternative embodiment, the disclosed system can test the user's lower-level brain functionality, such as vestibular brain functions, and, if the user's function is normal, can test the next level of the user's brain functionality, such as gross motor and cerebellum. It can continue working up to the test the higher-level functions until it determines if and where a user needs improvement. Then, when the system determines an area that needs improvement, the assessment portion of the disclosed system can be paused and the training portion can commence. Therefore, in this embodiment, instead of delivering a series of activities across all the functional areas targeted for improvement, the system can target activities for a single weak area until that area improves, and then move on to a new weak area at a higher brain functionality.

In one embodiment, the system may provide a person who has left vestibular, left cerebellum weakness with all of the available neutral and left deficit activities. The user can start at level one for all of these activities, train at level one for all activities, and, as the user improves in each activity, can then move on to the next level for the corresponding activities. In one embodiment, each activity has three levels of difficulty. Additionally, if the user is trained in an area, the improved functions can be integrated into an activity that trains other brain functions in order to improve overall performance. For example, if an individual has tested for improved balance, the system can present the individual with an activity that trains balance while simultaneously incorporating skills such as motor planning and visual tracking in order to achieve a better, longer lasting change. Additionally, the system can use color and sound to amplify the effectiveness of the activity. Once the lower-level brain functions have improved performance, overall brain capacity and performance can be improved for higher-level tasks.

In another embodiment, the system will start a user with training activities at level one for one brain functionality (for example, level one for all activities that train vestibular function). The user will move up through additional levels of those activities until each of those levels are completed. Once the user has completed all of the levels of all of the activities for one brain functionality (for example, vestibular functionality), the system will test the user on the next higher brain functionality (for example, gross motor and cerebellum). At this next functionality, if the assessment determines that the user requires training, the user will once again start at level one for all relevant activities (for example, gross motor and cerebellum) and work his or her way up through the levels until all levels of all activities relevant to that brain function are completed. Once all levels of all relevant activities are completed, the user will be tested, and possibly trained, on the next brain functionality.

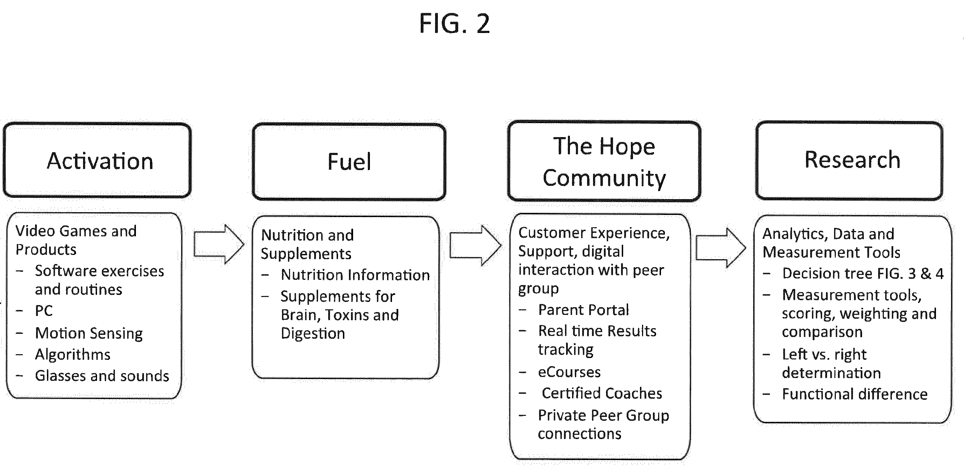

In a preferred embodiment, as illustrated in FIG. 2, the disclosed invention includes Bluetooth-enabled glasses, a dance mat, a camera, a computing system, a motion sensing system, and a system and method that includes brain function assessment capabilities, brain fitness training activities, data collection methods, data analysis methods, communication capabilities, internet capabilities, and an online community platform that can engage and offer support to users and provide nutritional information.

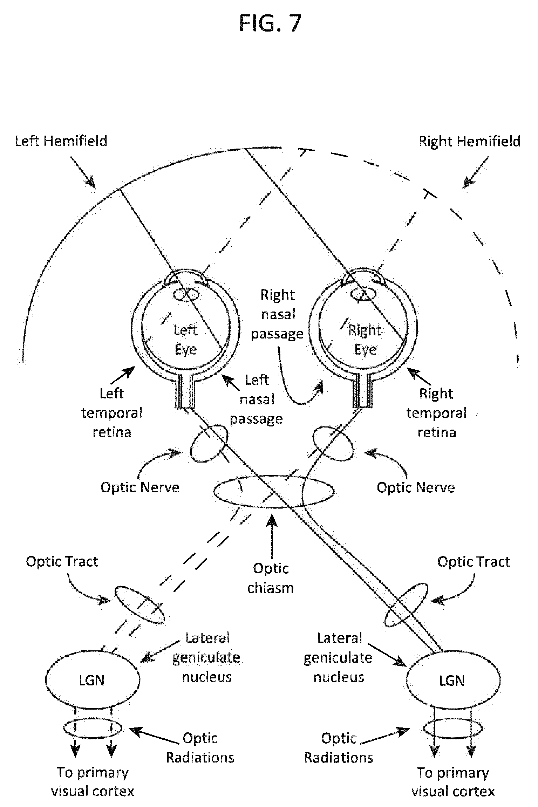

In general, visual input from either eye can send a signal to one hemisphere of the brain depending on which direction the visual input is coming from. For example, as illustrated in FIG. 7, visual input from a person's right side is directed to the left hemisphere of the brain and visual input from a person's left side is directed to the right hemisphere of the brain. Additionally, visual input from the top part of a person's vision stimulates the temporal lobe and visual input from the bottom part of a person's vision parietal the temporal lobe. Therefore, on screen elements can be used to improve or increase a person's brain functionality based on where they are located on a screen (ex: right, left, top, or bottom).

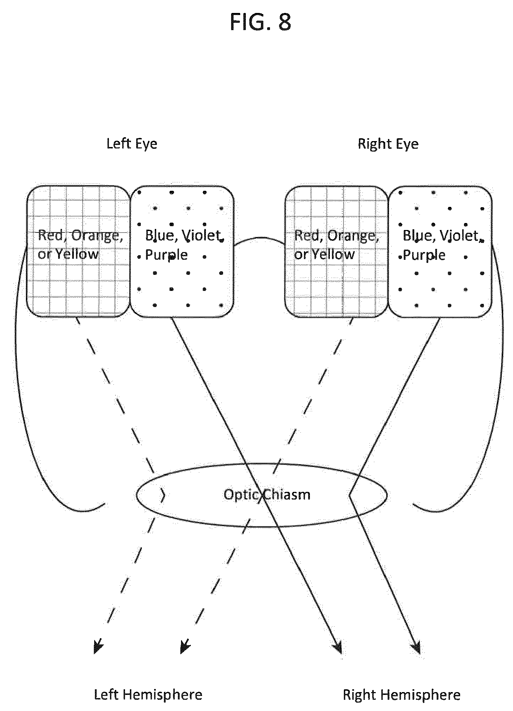

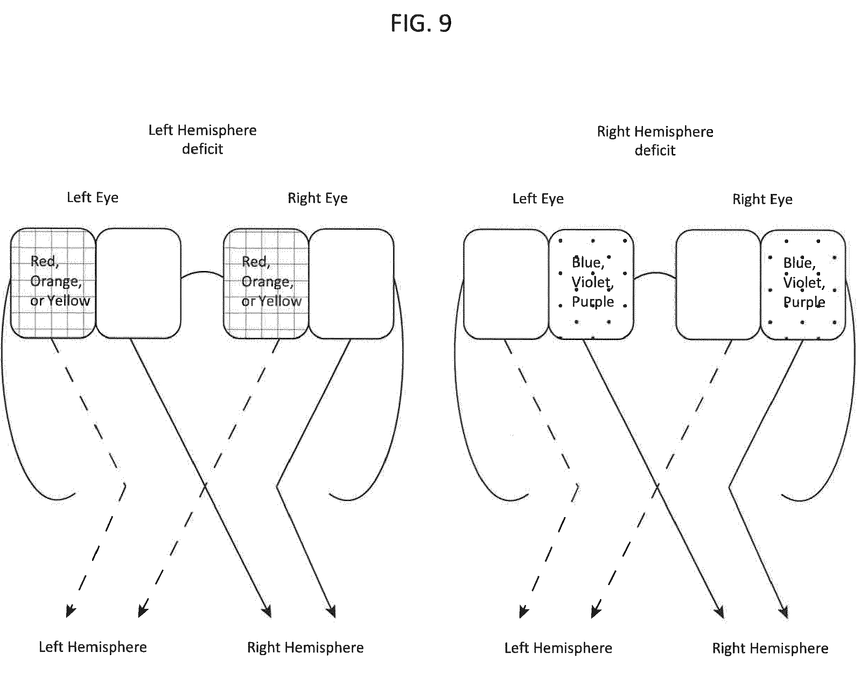

Additionally, color and sound are known to stimulate the brain. For example, red is known to stimulate the left hemisphere of the brain, while blue is known to stimulate the right hemisphere of the brain, as illustrated in FIGS. 8 and 9. Further, the left hemisphere of the brain is stimulated by high tones, while the right hemisphere of the brain is stimulated by low tones.

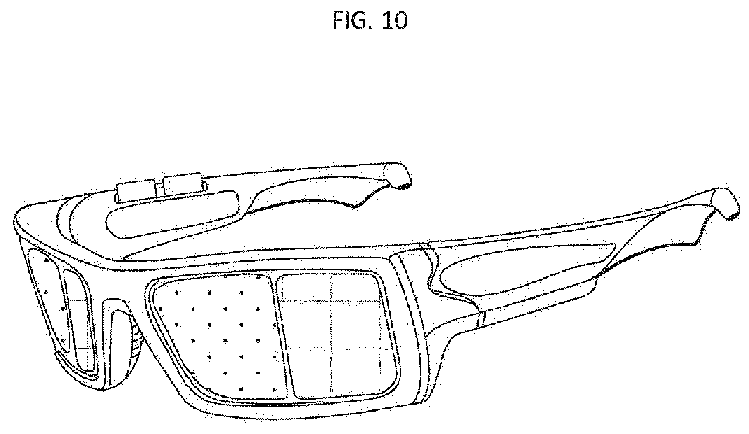

The Bluetooth-enabled glasses, as illustrated in FIG. 10 and disclosed herein, can be glasses with red/blue lenses. In one embodiment, the glasses can be standard size safety glasses with colored lenses. In a preferred embodiment, the left half of both lenses can be red and the right half of both lenses can be blue, as illustrated in FIGS. 8 and 10. In another embodiment, as illustrated in FIG. 9, one half of each lens can be blocked or black. For example, the left half of each lens may be red while the right half of each lens is black. This setup would help an individual with a left hemisphere deficit. In another example, the right half of each lens may be blue while the left half of each lens is black. This setup would help an individual with a right hemisphere deficit. In another embodiment, the lenses are easily removable from the glasses so that other lenses, such as clear or tinted lenses, can be replaced in the glasses. In a further embodiment, the color of the lenses can automatically change based on feedback provided by a software game. For example, if the game is aiming to stimulate the right hemisphere, the game will communicate to the lenses that they should turn blue, and the lenses can then turn blue.

Further, as illustrated in FIG. 10, the Bluetooth-enabled glasses can have earbuds built into the ends of the glasses in order to coordinate music play and further stimulate left and right brain functions, although the music play can be played using any audio system having speakers. The glasses can connect to any Bluetooth-enabled device. Therefore, the wearer can have simultaneous visual and auditory input when wearing the Bluetooth-enabled glasses.

For example, to stimulate the left hemisphere of the brain, the glasses or other speaker may play detailed, high frequency tones or music with lyrics and may primarily use the notes G, B, and A. On the other hand, to stimulate the right hemisphere, the glasses or other speaker may play low frequency music with no lyrics and may primarily use the notes C, D, E, and F. In order to play sound, in some embodiments, the glasses or other speaker may require a battery, such as at least one rechargeable or disposable battery.

In some embodiments, the auditory feature can be provided through the computing device or through a separate speaker. Regardless of how the sound reaches the user, the system will control the sound played so that the proper frequency and notes are played for the user's determined hemisphere weakness.

In addition to using the glasses for training, the system includes specific training activities. These training activities can train a brain function on their own, or they can be integrated with other training materials for a more effective result. For example, the system can train balance by having the user complete a physical activity, or it can train balance by combining a physical activity with visual or auditory input (for example, the glasses).

In one embodiment, the training activity used in the system involves use of a person's whole body, which can activate the user's brain across specific left and right hemispheric regions and can increase learning. These whole-body training activities can be hands-free and integrate motion, motor-sensory, rhythm and vision through the use of the Bluetooth glasses, a motion sensing system, and the system and method described herein. Some of the activities can incorporate visual/picture reading, spelling and math, additional sensory-motor, or hand-eye fine motor activities.

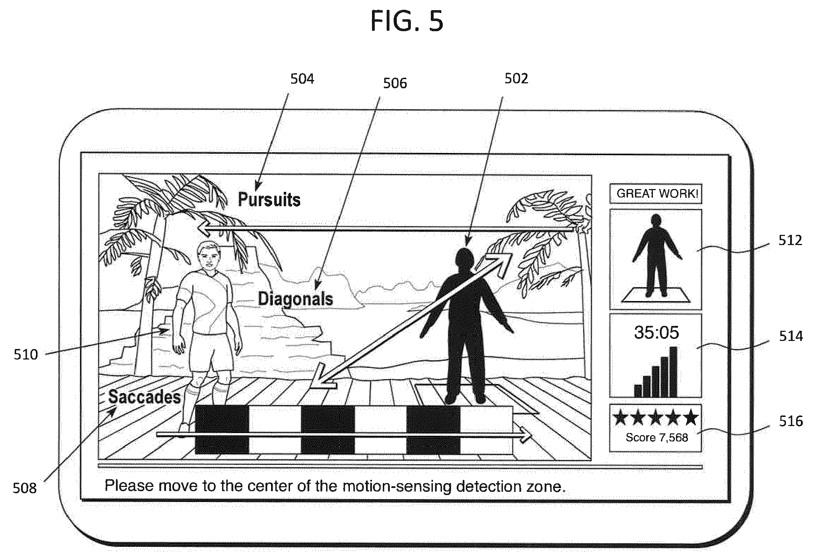

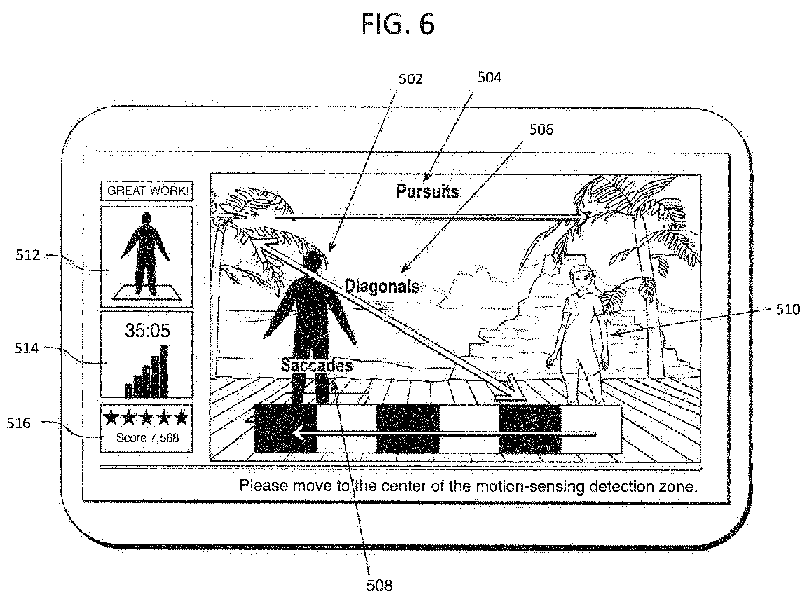

For example, as illustrated in FIGS. 5 and 6, one or more series of activities can be displayed to a user on a screen. More specifically, as illustrated in FIG. 5, a user with a left-brain weakness or deficit can complete right-handed and right-legged activities using an on screen avatar 502 that can be located on, for example, the right side of the screen. More specifically, objects displayed, such as horizontal pursuits 504, can move slowly from right to left, objects such as diagonal pursuits 506 can move in a diagonal pattern from the bottom left to the upper right portion of the screen, objects on the screen, such as trains, logs, or waves, otherwise known as saccades 508 or fast-moving objects, can move quickly from left to right, popups displayed may appear on the right side of the screen, and an activity that involves drawing or tracing objects may occur on the right side of the screen. Additionally, the user may listen to high frequency sounds or music during the activity, and the screen and on-screen elements may be primarily warm colors such as, but not limited to, red, yellow, and orange. To help the user, the display may show an on-screen instructor 510 that is used to illustrate desired movements to the user. Off to the side, as illustrated in FIG. 5, the display may show a second avatar 512 that is removed from the environment, a timer 514 to show time elapsed while completing the activity, and a score 516 so that the user knows how well he or she is accomplishing the activity.

For a user with a right-brain weakness or deficits, the user will complete left-handed and left-legged activities using an on screen avatar 502 that can be located on, for example, the left side of the screen, as illustrated in FIG. 6. More specifically, objects displayed, such as horizontal pursuits 504, can move slowly from left to right, objects such as diagonal pursuits 506 can move in a diagonal pattern from the bottom right to the upper left portions of the screen, objects on the screen, such as trains, logs, or waves, otherwise known as saccades 508 or fast-moving objects, can move quickly from right to left, popups displayed may appear on the left side of the screen, and an activity that involves drawing or tracing objects may occur on the left side of the screen. Further, the user may listen to low frequency sounds or music during the activity, and the screen and on-screen elements may be primarily cool colors such as, but not limited to, green, blue, navy, and purple. As with activities for left-brain weakness or deficits, to help the user, the display may show an on-screen instructor 510 that is used to illustrate desired movements to the user. Off to the side, as illustrated in FIG. 5, the display may show a second avatar 512 that is removed from the environment, a timer 514 to show time elapsed while completing the activity, and a score 516 so that the user knows how well he or she is accomplishing the activity.

Because the activity on screen can coordinate with the sound being played, a user can have an integrated learning experience. For example, to stimulate the right hemisphere, the activity on screen may have the user targeting left hand movements and left leg movements, the user may be looking through the Bluetooth-enabled glasses that have a partially blue lens, and the system may be playing low frequency music with no lyrics. However, while the auditory element can be integrated with another function such as vision (through the glasses) or motor planning (through physical activity), the auditory element has the option of being isolated and used as a solo training element.

In one embodiment, the motion sensing system used in coordination with the training activities can be comprised of a motion sensor for a gaming console, smart clothing that is motion trackable, or any other device that can track balance, head and body positions, 2D and 3D motions, and that can record data analytics to a back-end server.

The motion tracking software can have precision tracking tools that track fine motor activities such as, but not limited to, finger movements, nose movements, feet movement, etc. Additionally, it can track gross motor activities, visual motor activities, balance, and gravity to determine where the user is in space. When tracking gravity, the motion tracking software uses the user's body's center of mass to determine how the user rotates in gravity and space.

In some embodiments, if the user is using a gaming console, the gaming console can include a motion sensor that communicates with a handheld controller that may be embedded with custom firmware that controls the system operation. For example, the handheld controller, such as a Wii Remote^{™} for Nintendo Wii^{™} or the Move Motion Controller for PlayStation®, can sense an infrared light beam sent from the gaming console and can communicate back its position relative to the console using Bluetooth technology.

Alternatively, the motion sensor may not communicate with a handheld controller, but may be a motion sensor that can pick up on the user's movements using, for example, infrared laser light cameras. For example, the motion sensor may be a Windows Kinect^{™} for PC, a Windows Kinect^{™} for the Xbox, a Leap Motion Controller connected to an Apple® or Microsoft® device, or a mobile phone using a camera as a motion sensor.

In another embodiment, a holographic computer, such as the Microsoft® HoloLens, or a virtual reality headset, such as the Oculus Rift®, can be used to track a user's motions in combination with a training activity. The activities and moving objects could be projected onto the lens for a user and the user could move in reaction to what is displayed.

The system described herein can collect and record data based on the user's movement for each series of interactive activities and can input the data into the computing system. The system can further compare the user's movement data from each series of interactive activities, which are each weighted and scored, to a desired user-executed outcome for each of the corresponding interactive activities and can display the comparison to the user in the form of feedback, which can be immediate feedback. This comparison is used to determine when the user has successfully completed each series of interactive activities. Additionally, the individual games disclosed herein can be loaded onto the gaming console wirelessly or can be stored on a disk and loaded onto the gaming console through the disk reader.

The motion sensing system can be used with a computing system such as, but not limited to, a desktop computer, a laptop computer, a television, such as a smart TV, or a tablet, such as, but not limited to, a Microsoft Surface^{™}, iPad®, Samsung Galaxy®, or Google Nexus. In some embodiments, the training activities can be coordinated with the motion sensing technology to be implemented on, for example, a television, a personal computer, a surface projector, or a tablet. The recorded data, in some embodiments, can be accessible to a user, such as a parent, through a computer that is connected to the Internet.

One example of a basic, vestibular training activity used in the system involves near-far focusing. When completing this activity, a user will hold a thumb in front of his or her face, identify a faraway small object or have a faraway object on screen, and look back and forth at the thumb and object at a moderate pace, ideally getting a clear image each time. The motion sensing technology can tell the user when his or her head is out of position, can score the user based on head position, and can collect data surrounding the head position movement.

Another example of a basic, vestibular training activity used in the system involves creating figure eights with hands and feet. This activity also trains for gross motor/cerebellum and visual improvements. When completing this activity, a user will push a car around a sideways figure eight track a predetermined number of times (for example, three times). The motion sensing technology can track the user's activity and compare it to a desired user-executed outcome, which can be displayed to the user in the form of immediate feedback.

One example of a gross motor/cerebellum training activity used in the system involves one foot hopping. When completing this activity, a user will stand on one foot with the other leg in the air and bent at the knee. The user will then hop side-to-side, using the same leg, over a line on the screen a certain number of times. In one embodiment, there are different levels to the activity. For example, the first level could require the user to hop 10 times, the second level could require the user to hop 10 times with a metronome element of 60 beats per minute, and a third level could require the user to hop 10 times to an irregular beat, but in a pattern. In one embodiment, music can be played while the user is doing the activity and the screen color will be chosen based on which foot the user is hopping on. For example, for a left hemisphere deficient user, the instructions provided will be for the user to hop on his or her right foot, while listening to high frequency music and viewing a screen with the colors red, yellow, and orange. During the activity, the motion sensing technology can track and record accurate hops.

One example of a higher-level, visual and auditory memory activity used in the system involves identifying digit span. When completing this activity, a user will, while standing, view a certain number of digits on a screen for a certain number of seconds. The numbers will then be removed from the screen and the user will be asked to repeat the numbers in order or point to the screen to indicate which numbers came first, second, third, etc. In one embodiment, there are different levels to the activity. For example, in the first level, the user will view three digits for three seconds or four digits for four seconds. Once the user successfully completes the activities in the first level, the user can move on to the second level, wherein the user will view five digits for five seconds or six digits for six seconds. Once the user successfully completes the activities in the second level, the user can move on to the third level, wherein the user will view seven digits for seven seconds.

In one embodiment, data can be collected from the training activities and can be analyzed and used for research. The disclosed system can also include tracking and immediate feedback to a user completing the exercises by utilizing the motion-sensing camera for body, hand, feet, and facial recognition.

In one embodiment, the disclosed system is digital and downloadable. Users can customize and computerize plans for motor-sensory-based activities that can be tailored to a specific individual. The plan can be designed so that an individual, such as a parent, can monitor another individual, such as a child. The training activity can also contain an online reporting system that indicates how many minutes the user has spent doing the activity and can track the player's progression over time. In one embodiment, an online leader board may exist that can encourage competition and experience building.

The online community platform can include access to currently available activities, new activity releases, courses, webinars, a certified coaching program, and various types of support functions. The courses can include videos, written content, a posting board, controlled class comments, and connection to social media outlets. Content can be released lesson by lesson. The webinars can be enabled to accept questions prior to their release. The community platform can also have means to accept feedback.

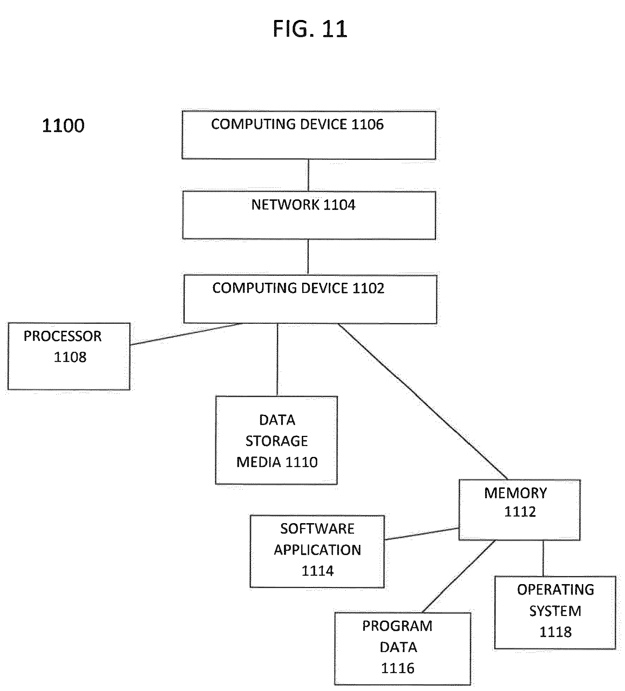

In some embodiments, the system described herein uses a computing system to carry out the various functions described herein. FIG. 11 is a schematic block diagram of an example computing system 1100. The example computing system 1100 includes at least one computing device 1102. In some embodiments the computing system 1100 further includes a communication network 1104 and one or more additional computing devices 1106 (such as a server).

The computing device 1102 can be, for example, be a computing device 1102 located in a user's home, school, or other place of business. In some embodiments, computing device 1102 is a mobile device. The computing device 1102 can be a stand-alone computing device or a networked computing device that communicates with one or more other computing devices 1106 across a network 1104. The additional computing device(s) 1106 can be, for example, located remotely from the first computing device 1102, but configured for data communication with the first computing device 1102 across a network 1104.

In some examples, the computing devices 1102 and 1106 include at least one processor or processing unit 1108 and system memory 1112. The processor 1108 is a device configured to process a set of instructions. In some embodiments, system memory 1112 may be a component of processor 1108; in other embodiments system memory 1112 is separate from the processor 1108. Depending on the exact configuration and type of computing device, the system memory 1112 may be volatile (such as RAM), non-volatile (such as ROM, flash memory, etc.) or some combination of the two. System memory 1112 typically includes an operating system 1118 suitable for controlling the operation of the computing device 1102, such as the WINDOWS® operating systems or the OS X operating system, or a server, such as Windows SharePoint Server. The system memory 1112 may also include one or more software applications 1114 and may include program data 1116.

The computing device 1102 may have additional features or functionality. For example, the computing device 1102 may also include additional data storage devices 1110 (removable and/or non-removable) such as, for example, magnetic disks, optical disks, or tape. Computer storage media 1110 may include volatile and nonvolatile, removable and non-removable media implemented in any method or technology for storage of information, such as computer readable instructions, data structures, program modules, or other data. System memory, removable storage, and non-removable storage are all examples of computer storage media. Computer storage media 1110 includes, but is not limited to, RAM, ROM, EEPROM, flash memory or other memory technology, CD-ROM, digital versatile disks (DVD) or other optical storage, magnetic cassettes, magnetic tape, magnetic disk storage or other magnetic storage devices, or any other medium which can be used to store the desired information and which can be accessed by the computing device 1102. An example of computer storage media 1110 is non-transitory media.

In some examples, one or more of the computing devices 1102 and 1106 can be located in an establishment. In other examples, the computing device 1102 can be a personal computing device, such as a personal computer or gaming device, that is networked to allow the user to access and utilize the system disclosed herein from a remote location, such as in a user's home, office or other location. In some embodiments, the computing device 1102 is a smart phone, tablet, laptop computer, smart TV, personal digital assistant, or other mobile device. In some embodiments, system operations and functions are stored as data instructions for a smart phone application. A network 1104 facilitates communication between the computing device 1102 and one or more servers, such as an additional computing device 1106, that hosts the system. The network 1104 may be a wide variety of different types of electronic communication networks. For example, the network 1104 may be a wide-area network, such as the Internet, a local-area network, a metropolitan-area network, or another type of electronic communication network. The network 1104 may include wired and/or wireless data links (such as through 3G or 4G networks). A variety of communications protocols may be used in the network 1104 including, but not limited to, Wi-Fi, Ethernet, Transport Control Protocol (TCP), Internet Protocol (IP), Hypertext Transfer Protocol (HTTP), SOAP, remote procedure call protocols, and/or other types of communications protocols.

In some examples, the additional computing device 1106 is a Web server. In this example, the first computing device 1102 includes a Web browser that communicates with the Web server to request and retrieve data. The data is then displayed to the user, such as by using a Web browser software application. In some embodiments, the various operations, methods, and functions disclosed herein are implemented by instructions stored in memory. When the instructions are executed by the processor 1108 of the one or more computing devices 1102 or 1106, the instructions cause the processor 1108 to perform one or more of the operations or methods disclosed herein.

The various embodiments described above are provided by way of illustration only and should not be construed to limit the claims attached hereto. Those skilled in the art will readily recognize various modifications and changes that may be made without following the example embodiments and applications illustrated and described herein and without departing from the true spirit and scope of the following claims.

## Claims

1. A functional learning system comprising:
a motion sensing system having precision tracking for fine motor activities, gross motor activities, visual motor activities, and balance tracking; and
a networked computing device having a processing device and a memory device, the memory device storing information that, when executed by the processing device, causes the processing device to:
assess a user's brain using a compared, weighting system to determine functional weaknesses in the user's brain;
determine a first series of interactive activities that targets the determined functional weaknesses;
display the first series of interactive activities to the user on a screen,
wherein the first series of interactive activities has a desired user-executed outcome and is weighted and scored;
collect data based on the user's completion of the first series of interactive activities;
compare the collected data from the user's completion of the first series of interactive activities to the desired user-executed outcome;
display the comparison as immediate feedback;
record the collected data to show measurable changes over time; and
determine when the user successfully completes the first series of interactive activities;
wherein:
when the processing device determines that the determined functional weakness is a right hemisphere brain weakness, pursuit objects are displayed on the screen and move slowly from left to right, and saccade objects are displayed on the screen and move quickly from right to left; and
when the processing device determines that the determined functional weakness is a left hemisphere brain weakness, pursuit objects are displayed on the screen and move slowly from right to left, and saccade objects are displayed on the screen and move quickly from left to right.

2. The system of claim 1, wherein, when the processing device determines that the user has successfully completed the first series of interactive activities, the processing device displays a second series of interactive activities to the user, and wherein the second series of interactive activities has a desired user-executed outcome and is weighted and scored.

3. The system of claim 1, wherein the first series of interactive activities instructs the user to use a left side of the user's body.

4. The system of claim 3, wherein pursuit objects are displayed on the screen and move between an upper left portion and a bottom right portion of the screen.

5. The system of claim 3, wherein the first series of interactive activities is displayed on a left side of the screen.

6. The system of claim 1, wherein, when the processing device determines that the determined functional weakness is a right hemisphere brain weakness, the displayed first series of interactive activities is displayed in colors selected from the group consisting of green, blue, purple, and combinations thereof.

7. The system of claim 1, wherein, when the processing device determines that the determined functional weakness is a right hemisphere brain weakness, the system simultaneously plays low frequency sounds.

8. The system of claim 1, wherein the first series of interactive activities instructs the user to use a right side of the user's body.

9. The system of claim 8, wherein pursuit objects are displayed on the screen and move between an upper right and a bottom left portion of the screen.

10. The system of claim 8, wherein the first series of interactive activities is displayed on a right side of the screen.

11. The system of claim 1, wherein, when the processing device determines that the determined functional weakness is a left hemisphere brain weakness, the displayed first series of interactive activities is displayed in colors selected from the group consisting of red, orange, yellow, and combinations thereof.

12. The system of claim 1, wherein, when the processing device determines that the determined functional weakness is a left hemisphere brain weakness, the system simultaneously plays high frequency sounds.

13. The system of claim 1, wherein the networked computing device syncs to Bluetooth-enabled glasses, the Bluetooth-enabled glasses comprised of a left lens and a right lens, wherein a left half of the left lens and a left half of the right lens is red and a right half of the left lens and a right half of a right lens is blue.

14. The system of claim 13, wherein the Bluetooth-enabled glasses are further comprised of earbuds enabled to play sound and stimulate predetermined regions of the brain.

15. The system of claim 1, wherein the motion sensing system additionally has precision tracking for the user's center of mass and its rotation in gravity and space.
